(19) Europäisches Patentamt
European Patent Office
Office européen des brevets

(11) **EP 4 276 167 A1**

(12) **EUROPEAN PATENT APPLICATION**
published in accordance with Art. 153(4) EPC

(43) Date of publication:
**15.11.2023 Bulletin 2023/46**

(21) Application number: **22736775.2**

(22) Date of filing: **07.01.2022**

(51) International Patent Classification (IPC):
*C12M 1/00* (2006.01)     *C12M 1/34* (2006.01)
*C12M 3/00* (2006.01)     *C12N 5/10* (2006.01)
*C12Q 1/04* (2006.01)     *C12Q 1/32* (2006.01)

(52) Cooperative Patent Classification (CPC):
**C12M 1/00; C12M 1/34; C12M 3/00; C12N 5/10;
C12Q 1/04; C12Q 1/32**

(86) International application number:
**PCT/JP2022/000292**

(87) International publication number:
**WO 2022/149607 (14.07.2022 Gazette 2022/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.01.2021 JP 2021001262**

(71) Applicant: **FUJIFILM Corporation
Tokyo 106-8620 (JP)**

(72) Inventors:
• **KUROSAWA, Takashi
Ashigarakami-gun, Kanagawa 258-8577 (JP)**
• **KOIKE, Makoto
Ashigarakami-gun, Kanagawa 258-8577 (JP)**
• **NAKAI, Shinichi
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54) **CELL SELECTION METHOD, DEVICE AND DEVICE SYSTEM**

(57)     An object of the present invention is to provide a method for selecting cells, an apparatus for selecting cells, and an apparatus system for selecting cells, which enables evaluation of a damage resistance of the cells in a suspension culture system. According to the present invention, provided is a method for selecting cells including introducing cells into a micro flow passage and allowing the cells to pass through the micro flow passage, evaluating a damage resistance of the cells to damage received by the cells due to passing through the micro flow passage, and selecting the cells based on the evaluation of the damage resistance.

FIG. 1

**Description**

Technical Field

The present invention relates to a method for selecting cells suitable for a mass production process, an apparatus for selecting cells, and an apparatus system for selecting cells.

Background Art

Conventionally, in the field of antibody pharmaceuticals, in selecting a clone suitable for a mass production process, a clone is selected using an amount of antibodies produced from cells as an index. On the other hand, in recent years, in order to increase the productivity of an antibody, a perfusion culture process in which cells are cultured at a high cell density for a long term has attracted attention. Perfusion culture with a high cell density is a culturing method that requires high stirring and a high oxygen supply amount, and the damage that the cells receive from the fluid in the culture vessel is larger than that in the conventional fed batch. Therefore, in a case where a clone selected using an amount of antibodies produced from cells as an index is applied to perfusion culture as conventionally, cell death may be caused by the damage from the fluid.

With the above circumstances, in selecting cells suitable for perfusion culture, it is desired to select a clone having high damage resistance in advance. For example, in Patent document 1, a fluid is allowed to flow through a chamber in which cells are immobilized, to apply a shear stress, and a response thereof is evaluated to determine stirring conditions. In addition, in Non-Patent Document 1, a cell suspension is allowed to flow through a micro flow passage to apply shear stress, and cell damage in the centrifugation step is evaluated.

Prior Art Documents

Non-Patent Documents

Non-Patent Document 1: Adrian Joseph et al., Biotechnol. J. 2017, 12, 1600730

Patent Documents

Patent Document 1: JP2016-52335A

**SUMMARY OF THE INVENTION**

In Patent Document 1, the cells are in a floating state in the culture, but the evaluation is performed in a state where the cells are adhered. In addition, in Non-Patent Document 1, it is a technique related to a study of conditions for centrifugation, not a technique related to cell selection.

An object of the present invention to be solved is to provide a method for selecting cells that enables evaluation of damage resistance of cells in a suspension culture system. Furthermore, an object of the present invention to be solved is to provide an apparatus for selecting cells and an apparatus system for selecting cells for performing the above-described method for selecting cells.

As a result of diligent studies to solve the above-described problems, the present inventor succeeded in evaluating the damage resistance of cells in the suspension culture system by introducing cells into the micro flow passage, allowing the cells to pass through the micro flow passage, and evaluating the damage resistance of cells to damage of cells received by passing the cells through the micro flow passage. The present invention has been completed based on the above findings.

That is, according to an aspect of the present invention, the following invention is provided.

<1> A method for selecting cells, including introducing cells into a micro flow passage and allowing the cells to pass through the micro flow passage,

evaluating damage resistance of the cells to damage received by the cells due to passing through the micro flow passage, and

selecting cells based on the evaluation of the damage resistance.

<2> The method according to <1>, in which the damage received by the cell is stress received from a fluid.

<3> The method according to <1> or <2>, in which the damage resistance of the cell is evaluated based on a damage state of the cell.

<4> The method according to any one of <1> to <3>, in which the damage resistance is evaluated based on viability with respect to an energy dissipation rate.

<5> The method according to any one of <1> to <3>, in which the damage resistance is evaluated based on a lactate dehydrogenase release rate of the cell with respect to an energy dissipation rate.

<6> The method according to any one of <1> to <3>, in which the damage resistance is evaluated based on viability against shear stress.

<7> The method according to any one of <1> to <3>, in which the damage resistance is evaluated based on a lactate dehydrogenase release rate of the cell against shear stress.

<8> The method according to any one of <1> to <7>, in which a plurality of the micro flow passages are used, and the micro flow passages are exchanged to evaluate the damage resistance of the cell.

<9> The method according to <8>, in which an inner diameter dimensional tolerance in the plurality of the micro flow passages is less than ±10%.

<10> The method according to any one of <1> to <9>, in which the micro flow passage is an electroforming tube.

<11> The method according to any one of <1> to <10>, in which an inner diameter of the micro flow passage is 10 $\mu$m to 3000 $\mu$m.

<12> The method according to any one of <1> to <7>, in which the micro flow passage is a groove.

<13> The method according to <12>, in which the groove consists of a combination of a metal plate and a metal plate with a groove.

<14> The method according to <12> or <13>, in which a width of each side of a cross section of the groove is 10 $\mu$m to 3000 $\mu$m.

<15> The method according to any one of <1> to <14>, in which the cell is a Chinese hamster ovary-derived cell or a human embryonic kidney cell 293.

<16> The method according to any one of <1> to <15>, in which the cells to be selected are a cell for producing a protein.

<17> The method according to any one of <1> to <16>, in which the cells are cells obtained by increasing the number of monoclonal cells.

<18> The method according to <16> or <17>, in which the cell for producing a protein is a cell for producing a protein by perfusion culture.

<19> A apparatus for selecting cells, including

a micro flow passage,
a pressure gauge for measuring a pressure upstream of the micro flow passage,
a first pump for feeding a cell suspension, which is provided upstream of the micro flow passage,
a second pump for feeding a cleaning liquid, which is provided upstream of the micro flow passage,
a pipe for supplying the cell suspension to the first pump and a pipe for supplying the cleaning liquid to the second pump,
a pipe for supplying a liquid from the first pump and a liquid from the second pump to the micro flow passage, and
a pipe for collecting liquid discharged from the micro flow passage.

<20> An apparatus system for selecting cells, including the apparatus for selecting cells according to < 19>, and a plurality of micro flow passages for being exchangeably used.

<21> The apparatus system according to <20>, in which an inner diameter dimensional tolerance in the plurality of the micro flow passages is less than ±10%.

According to the method for selecting cells, the apparatus for selecting cells, and the apparatus system for selecting cells according to the present invention, the damage resistance between cells in a floating system can be quantified. Furthermore, the viability and the proliferation rate of the cells during culture can be predicted from the damage resistance index, and can be used as an index for selecting the cells.

## BRIEF DESCRIPTION OF THE DRAWINGS

Fig. 1 shows an example of the apparatus of the present invention.
Fig. 2 shows an example of a method for producing a divided flow passage in which a metal plate and a metal plate with a groove are combined.
Fig. 3 shows the results of measuring the variation in pipe diameter of a Steel Use Stainless (SUS) pipe and an electroforming pipe.
Fig. 4 shows the results of measuring the repetitive reproducibility of the SUS pipe and the electroforming pipe.
Fig. 5 shows the results of performing quantification of damage resistance evaluation values twice for three types of clones.
Fig. 6 shows a cell culture apparatus used in perfusion culture.
Fig. 7 shows measurement results of the viability in perfusion culture.
Fig. 8 shows the measurement results of the bleeding rate in perfusion culture.
Fig. 9 shows the results of performing quantification of damage resistance evaluation values in human embryonic kidney cells 293.

**DESCRIPTION OF THE PREFERRED EMBODIMENTS**

Hereinafter, the contents of the present invention will be described in detail.

<Method for selecting cells>

The method for selecting the cells according to the embodiment of the present invention is a method including

introducing cells into a micro flow passage and allowing the cells to pass through the micro flow passage,
evaluating damage resistance of the cells to damage received by the cells due to passing through the micro flow passage, and
selecting cells based on the evaluation of the damage resistance.

In the present invention, in a case of evaluating the damage resistance of the cell, quantifying the damage resistance using the physical property value of the cell culture solution is preferable. The damage resistance of the cell can preferably be evaluated based on the damage state of the cell.
As an example, the cell density after receiving the damage can be compared with the cell density before receiving the damage. That is, the cell density after passing through the micro flow passage can be compared with the cell density before passing through the micro flow passage. For the cells before receiving the damage and the cells after receiving the damage, the damage resistances of the cell can be evaluated using the substance amount of the component discharged from the cells. Particularly preferably, the damage resistance of cell can be evaluated using dead cell density, viable cell density, viability, lactate dehydrogenase (LDH) concentration, host cell-derived protein (HCP) concentration, DNA concentration, turbidity, or the like, as an index.
In the present invention, the Reynolds number (Re) [-], the density ($\rho$) [kg/m$^3$], the flow velocity (u) [m/s], the pipe inner diameter (d) [m], the viscosity ($\mu$) [Pa·s], the flow rate (U) [m$^3$/s], the energy dissipation rate (EDR) [W/m$^3$], the pressure loss ($\Delta$P) [Pa], the pipe length (1) [m], and the loading time (t) [s] have the following relationship.

$$Re = (\rho \times u \times d)/\mu$$

$$u = 4U/(\pi d^2)$$

$$EDR = (d^2 \times \Delta P^2)/(16 \times 1^2 \times \mu)$$

$$t = (\pi \times d^2 \times 1)/(4U)$$

In the present invention, the energy dissipation rate (EDR) is preferably 10 to $10^{11}$ [W/m$^3$], more preferably $10^5$ to $10^{11}$ [W/m$^3$], and particularly preferably $10^5$ to $10^{10}$ [W/m$^3$] of the lethal region of CHO cells. Most preferably, EDR is $2.0 \times 10^9$ to $3.0 \times 10^9$ [W/m$^3$]. The energy dissipation rate can be used as an index of damage given to cells. In the present specification, the description of "A to B" representing a numerical range means a numerical range including a lower limit and an upper limit given as an end points, unless otherwise specified.
Re is defined to be Re $\leq$ 2000, which is a laminar flow region.
The time for loading the damage (loading time (t)) is preferably $1.0 \times 10^{-7}$ seconds to 100 seconds, more preferably $1.0 \times 10^{-6}$ seconds to 50 seconds, and still more preferably $1.0 \times 10^{-5}$ seconds to 10 seconds.
The present disclosure can be used for selecting a cell clone for use in a culturing method, in which the energy dissipation rate is preferably 10 to $10^{11}$ [W/m$^3$], more preferably $10^5$ to $10^{11}$ [W/m$^3$], particularly preferably $10^5$ to $10^{10}$ [W/m$^3$], and most preferably $2.0 \times 10^9$ to $3.0 \times 10^9$ [W/m$^3$].

In the present invention, the damage resistance can be preferably evaluated based on the viability with respect to the energy dissipation rate. The energy dissipation rate is an index of the magnitude of cell damage due to the fluid. The larger the energy dissipation rate is, the larger the cell damage is, and the lower the viability is. By allowing the cell to pass through the micro flow passage, the cell passes through a space having a certain energy dissipation rate, and damage is applied to the cell. The viability with respect to the energy dissipation rate means the viability after the cell has passed through a space having a certain energy dissipation rate.

The energy dissipation rate when passing through the micro flow passage can be obtained by measuring the pipe inner diameter, the viscosity, the pipe length, and the pressure loss. Although the viability can be measured by a known method, it is preferable to measure the viability using Vi Cell XR.

In the present invention, the damage resistance can be preferably evaluated based on the lactate dehydrogenase release rate of the cell with respect to the energy dissipation rate. The lactate dehydrogenase release rate with respect to the energy dissipation rate means the lactate dehydrogenase release rate after the cells have passed through a space having a certain energy dissipation rate.

The damage received by the cell is preferably the stress received from the fluid.

The motion of cells in a fluid can be classified into four basic elements: translational motion, expansion and contraction, shear deformation, and rotation. Among the above, expansion and contraction and shear deformation are motions that deform cells.

Among expansion and contraction, the expansion is the deformation in a case where the flow velocity $u_x$ in the flow axis x direction increases in the x direction ($\delta u_x / \delta x > 0$), and the contraction is the deformation in a case where the flow velocity $u_x$ in the flow axis x direction decreases in the x direction ($\delta u_x / \delta x < 0$).

The shear deformation is the deformation in a case where the flow velocity $u_x$ in the flow axis x direction changes in the y direction ($\delta u_x / \delta y \neq 0$).

Under complex fluid motion in the culture vessel, the cells are under stress of expansion, contraction, and shear.

As described above, examples of the damage include expansion and contraction and shear stress.

Preferably, the damage is a shear stress.

In the present invention, the damage resistance can be preferably evaluated based on the viability against shear stress. The shear stress is an index of the magnitude of cell damage due to the fluid, and the larger the shear stress is, the lower the viability is.

The shear stress can be calculated from the energy dissipation rate and the viscosity of the cell suspension. The viability against shear stress means the viability after the cell has passed through a space having a certain shear stress.

The shear stress when passing through the micro flow passage can be obtained by measuring the pipe inner diameter, the viscosity, the pipe length, and the pressure loss. In this case, the viability can be measured by a known method, but it is preferably measured using Vi Cell XR.

The shear stress ($\tau$) has the following relationship between the viscosity ($\mu$) and the change in the flow axis x-direction flow velocity $u_x$ in the y direction ($\delta u_x / \delta y$).

$$\text{Shear stress } (\tau) \text{ [Pa]} = \mu \times (\delta u_x / \delta y)$$

In the present invention, the shear stress ($\tau$) is preferably $1.0 \times 10^{-1}$ to $1.2 \times 10^4$ [Pa], more preferably $1.0 \times 10^1$ to $1.2 \times 10^4$ [Pa], and particularly preferably $1.0 \times 10^1$ to $3.6 \times 10^3$ [Pa] of the lethal region of the CHO cells. Most preferably, the shear stress is $1.5 \times 10^3$ to $1.9 \times 10^3$ [Pa].

In the present invention, the damage resistance can be preferably evaluated based on the lactate dehydrogenase release rate of the cell against the shear stress.

The form of the micro flow passage is not particularly limited, and for example, a tube (electroforming tube) having a small inner diameter tolerance produced by electroforming, a divided flow passage in which a metal plate and a metal plate with a groove are combined to be disassemblable and cleanable, or the like can be used. In the case of the divided flow passage, the micro flow passage is a groove.

In a case where the micro flow passage is an electroforming tube, an inner diameter of the electroforming tube is generally 10 μm to 3000 μm, preferably 20 μm to 2000 μm, more preferably 30 μm to 1000 μm, still more preferably 40 μm to 500 μm, and particularly preferably 50 μm to 300 μm.

In a case where the micro flow passage is an electroforming tube, the cross section of the flow passage is preferably circular. In addition, in the present specification, the electroforming tube and the electroforming pipe mean the same thing.

In a case where the micro flow passage is a groove, a width of each side of the cross section of the groove is

generally 10 μm to 3000 μm, preferably 20 μm to 2000 μm, more preferably 30 μm to 1000 μm, still more preferably 40 μm to 500 μm, and particularly preferably 50 μm to 300 μm.

The length of the micro flow passage is generally 10 mm to 2000 mm, preferably 20 mm to 500 mm, more preferably 30 mm to 100 mm, and particularly preferably 40 mm to 60 mm.

In the present invention, the damage resistance of cells can be evaluated by using a plurality of micro flow passages and exchanging the micro flow passages.

In a case where a plurality of micro flow passages are used as described above, the inner diameter dimensional tolerance in the plurality of micro flow passages is preferably less than ±10%, more preferably ±8% or less, still more preferably ±5% or less, and particularly preferably ±3% or less.

By lowering the inner diameter dimensional tolerance, the reproducibility of the damage resistance evaluation can be improved.

In a case of performing evaluation between a plurality of clones in the antibody manufacture, it is preferable that there are a plurality of micro flow passages because clogging occurs in one micro flow passage. As the clone to be evaluated, there may be 3 or more clones, 10 or more clones, or 100 or more clones. In addition, as the selected clone having high damage resistance, there may be 3 clones or less, 5 clones or less, 10 clones or less, or 50 clones or less.

The type of the cell in the present invention is not particularly limited, but is preferably an animal cell and more preferably a mammalian cell. The cell may be a primary cell or a strained cell. The cell may be a genetically engineered cell (for example, a cell into which a gene has been introduced from the outside).

Examples of the cell include a Chinese hamster ovary-derived cell (CHO cell), a human embryonic kidney cell 293 (also referred to as HEK293), a monkey cell COS cell, a rat myeloma cell, a mouse myeloma cell, but the present invention is not particularly limited thereto. These cells may be a cell into which a foreign gene encoding a protein to be expressed is introduced. Other examples of the cell include stem cells such as induced pluripotent stem cell (iPS) or mesenchymal stem cell (MSC). The cell is preferably a Chinese hamster ovary-derived cell or a human embryonic kidney cell 293, and more preferably a Chinese hamster ovary-derived cell.

An expression vector can be used for introducing a foreign gene encoding a protein to be expressed into a cell. A cell into which a foreign gene encoding a protein to be expressed is introduced can be produced by introducing an expression vector containing DNA encoding the protein to be expressed, an expression control sequence (for example, an enhancer, a promoter, a terminator, and the like), and optionally a selection marker gene, into the cell. The expression vector is not particularly limited, and can be appropriately selected and used depending on the type of the cell, use, or the like.

As the promoter, any promoter can be used as long as it can exert a function in mammalian cells. Examples thereof include an immediate early (IE) gene promoter of cytomegalovirus (CMV), an initial promoter of SV40, a retrovirus promoter, a metallothionein promoter, a heat shock promoter, an SRα promoter, a promoter of moloney murine leukemia virus, and an enhancer. In addition, an enhancer of the IE gene of human CMV may be used together with the promoter.

As the selection marker gene, for example, a drug resistance gene (a neomycin resistance gene, a DHFR gene, a puromycin resistance gene, a blastcidin resistance gene, a hygromycin resistance gene, a cycloheximide resistance gene), a fluorescence gene (a gene encoding a green fluorescent protein GFP or the like), or the like can be used.

The method for introducing the expression vector into the cell is not particularly limited, and for example, a calcium phosphate method, an electroporation method, a liposome method, a gene gun method, a lipofection method, or the like can be used.

After introducing DNA encoding the protein to be expressed in a cell and a selection marker gene, a plurality of monoclonal cells expressing the target protein can be selected by a known method.

The present disclosure can be used to select a clone suitable for suspension culture from a plurality of monoclonal clones. Particularly, it is preferably used for selecting a clone suitable for perfusion culture.

In the present disclosure, using some cells from a plurality of monoclonal cell clones, the damage resistance of each cell can be evaluated by allowing each cell to pass through a micro flow passage. Then, the damage of the cells is evaluated, and for example, a clone having high damage resistance can be selected and used for long-term culture. In a case of selecting a clone having high damage resistance, a clone to be used for culture can be selected in combination with another evaluation method can be combined to select. In addition, it can be selected in combination with another evaluation method after selecting a clone having high damage resistance. The other evaluation method is not limited as long as it is a known selection method, and selection can also be performed by combining protein production efficiency or gene expression as an index. As the culture, perfusion culture is preferable.

In the present invention, by selecting cells based on the evaluation of damage resistance, cells that can tolerate long-term culture can be preferably selected. In a case of selecting a cell for manufacturing a target protein, a cell that can tolerate long-term culture is selected, and the target protein is produced using this cell to produce a large amount of protein.

The cell for manufacturing the target protein described above is preferably a cell for manufacturing the protein by perfusion culture.

In the present invention, a cell having high perfusion culture tolerance can be selected based on the damage resistance evaluated by the method according to the embodiment of the present invention. As the cell having high perfusion culture tolerance, a cell having high viability and high bleeding rate (index of cell proliferation rate) in perfusion culture can be selected.

From the viewpoint of reproducibility, the viability after the damage loading has a small value of coefficient of variation (CV) or a small standard deviation. The CV value is preferably 10% or less and more preferably 5% or less, and it is particularly preferable that the viability can be evaluated by the CV value of 3% or less.

In addition, in the present invention, the threshold value of the resistance index can be determined from the comparison between the measurement result of the resistance index and the result of the culture of each cell.

In addition, in the present invention, a cell clone having a high survival rate after being loaded with damage to the cell by the apparatus for selecting cells according to the embodiment of the present invention can be selected as the cell clone having a high resistance to damage.

In addition, in the present invention, cell culture conditions (stirring blade diameter, stirring blade height, number of stirring blades, stirring rotation speed, various gas aeration volumes, various bubble diameters) can be determined from the resistance index.

<Apparatus and apparatus system for selecting cells>

The apparatus for selecting cells according to the embodiment of the present invention includes

a micro flow passage,
a pressure gauge for measuring a pressure upstream of the micro flow passage,
a first pump for feeding a cell suspension, which is provided upstream of the micro flow passage,
a second pump for feeding a cleaning liquid, which is provided upstream of the micro flow passage,
a pipe for supplying the cell suspension to the first pump and a pipe for supplying a cleaning liquid to the second pump,
a pipe for supplying a liquid from the first pump and a liquid from the second pump to the micro flow passage, and
a pipe for collecting liquid discharged from the micro flow passage.

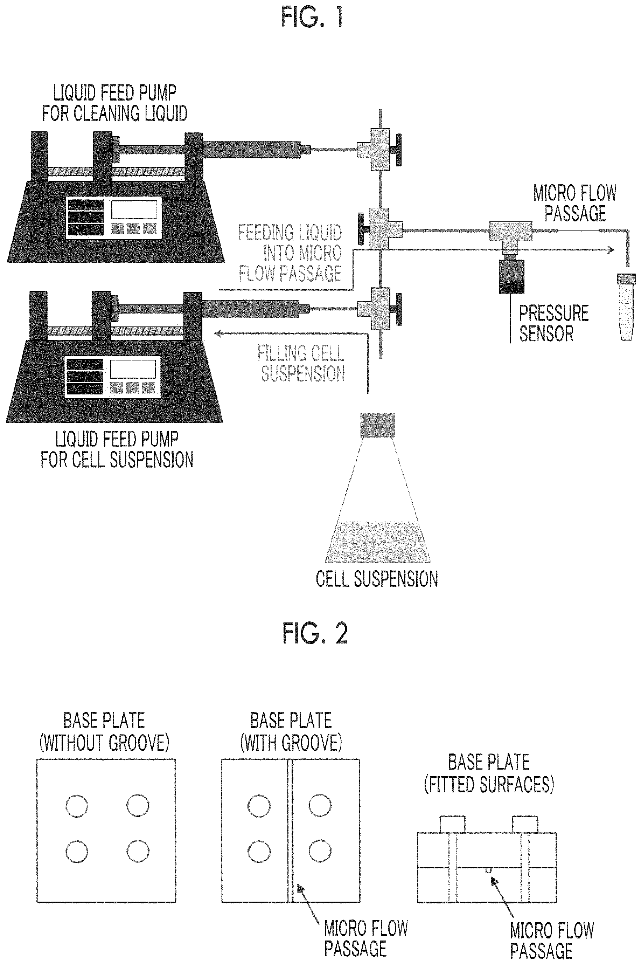

An example of the apparatus of the embodiment of the present invention is shown in Fig. 1.

The micro flow passage is a flow passage for applying a physical stress to the cell.

The details of the micro flow passage are as described above in the present specification.

As described above in the present specification, it is preferable that the micro flow passage is used by passing cells once or a plurality of times, the used micro flow passage is then removed, a new micro flow passage is provided, and a new measurement is performed. In this case, a plurality of micro flow passages are used. Therefore, by further combining a plurality of micro flow passages for exchange and use with the apparatus for selecting cells according to the embodiment of the present invention, the apparatus system for selecting cells can be provided.

A method for producing a tube by electroforming (electronical casting) is a method for manufacturing a metal product by an electroplating method. A metal is precipitated on the matrix to a desired thickness and electrodeposited, and then the electrodeposition layer is peeled off from the matrix to obtain an electrodeposited product. A fine tube can be manufactured by using the ultrafine metal wire as the matrix, electrodepositing to a desired thickness, and then removing the ultrafine metal wire.

An example of a method for producing a divided flow passage in which a metal plate and a metal plate with a groove are combined to be disassemblable and cleanable will be described below with reference to Fig. 2.

As the base plate, two SUS materials having a size of 50 mm square and a thickness of 15 mm are used (one without a groove and one with a groove). For a base plate used as a grooved base plate, a groove having a width of 0.12 mm, a depth of 0.12 mm, and a length of 50 mm is formed. The surfaces of the two base plates are fitted, and four screws are tightened to provide a mechanism for sealing with a metal touch (Fig. 2).

The pressure gauge is a meter that measures a pressure value for quantifying the physical stress applied to a cell. The pressure gauge is not particularly limited as long as it can measure the pressure upstream of the micro flow passage, and a commercially available pressure gauge can be used. The pressure gauge can be provided close to the upstream of the micro flow passage such that the pressure loss in the micro flow passage can be estimated. By providing the pressure gauge close to the upstream of the micro flow passage, it can be considered that the pressure in the micro flow passage is measured.

As the pump, a first pump for feeding the cell suspension and a second pump for feeding the cleaning liquid are provided. Each of the first pump and the second pump is provided upstream of the micro flow passage.

As the pipes, a pipe for supplying the cell suspension to the first pump, a pipe for supplying the cleaning liquid to the second pump, a pipe for supplying a liquid from the first pump and a liquid from the second pump to the micro flow passage, a pipe for collecting the liquid discharged from the micro flow passage, and the like can be provided.

The present invention will be described in more detail with reference to the following examples, but the present invention is not limited to the examples.

Examples

Example 1: Evaluation of damage resistance

<1> Procedure for evaluating damage resistance

<1-1> Preparation before damage resistance evaluation

By constructing a vector containing nucleic acid sequences encoding IgG1 and IgG4 and introducing the constructed vector into a CHO-DG44 cell, the CHO-DG44 cell expressing IgG1 (IgG1 cell) and the CHO-DG44 cell expressing IgG4 (IgG4 cell) were produced. Construction of the vector and introduction into the cell were performed according to Example 2 of JP2016-517691A. Based on the above, three types of monoclonal clones collected from different colonies were prepared using CHO cells producing a monoclonal antibody.
A human embryonic kidney cell 293 (HEK293) was purchased from ATCC.
For the CHO cell, the damage resistance of each of the cells subcultured 4 times in a conical flask was evaluated. Prior to performing the damage resistance evaluation, the cell density of each clone was adjusted to $1.5 \times 10^6$ cells/mL by a dilution operation. The diluted cell suspension was heated to 37°C in an incubator, and then the damage resistance was evaluated.
the damage resistance of each of the HEK293 cells subcultured 4 times in a conical flask was also evaluated in the same manner as for CHO cells. Prior to performing the damage resistance evaluation, the cell density of the clones was adjusted to $1.5 \times 10^6$ cells/mL by a dilution operation. The diluted cell suspension was heated to 37°C in an incubator, and then the damage resistance was evaluated.

<1-2> Damage resistance evaluation apparatus (Fig. 1)

As the liquid feed pump, a syringe pump (PHD ultra 4400) manufactured by Harvard Apparatus was used, and two pumps, a pump for feeding the cell suspension and a pump for feeding the cleaning liquid, were used. A pressure gauge (AP-14S manufactured by KEYENCE CORPORATION) for quantifying EDR was disposed on an upstream side (inlet side) of the micro flow passage. For the micro flow passage, a circular tube having an inner diameter of 0.1 mm (dimensional tolerance of ±2%) and a length of 50 mm, manufactured by electroforming was used.
For the damage load on the cells, the cell suspension is sucked with a syringe, and then the valve is switched to feed the cell suspension to the micro flow passage (liquid feeding rate: 7.7 mL/min). EDR is estimated from the pressure value during liquid feeding, and it is confirmed that the EDR is within the range of 2.20 to $2.30 \times 10^9$ [W/m³]. In a case where the EDR value deviates from the above, the micro flow passage is replaced with a new one.

<1-3> Quantification of damage resistance evaluation value

For sampling of the sample, about twice the volume of the pipe or the sensor part is first discarded, and the actual sampling is performed.

(Measuring method for viability)

The viability is a ratio of the viable cell densities before and after the damage is loaded, and is presented by the following equation.
Before the damage loading is a state before the cells are fed to the micro flow passages, and after damage loading is a state after the cells are fed to the micro flow passages.

$$\text{Viability } [\%] = \frac{\text{Viable cell density after damage loading [Mcells/ml]}}{\text{Viable cell density before damage loading [Mcells/ml]}}$$

The viable cell density was measured with a ViCell XR manufactured by Beckman Coulter Life Sciences.
The detection condition of the particles was 6 to 50 [μm], and the number of images was 50.

(Measuring method for energy dissipation rate)

The energy dissipation rate is obtained by substituting the pipe inner diameter (d), the viscosity of the cell suspension (μ), the pipe length (1), and the actually measured pressure loss ΔP into the following equation. The pressure loss ΔP is a differential pressure between the pressure on the primary side (inlet side) of the micro flow passage and the secondary side (outlet side) of the micro flow passage. Since the secondary side (outlet side) of the micro flow passage is atmospheric pressure, it was set to 0. Therefore, the pressure loss ΔP was set to be the same as the actually measured primary side pressure.

$$\text{Energy dissipation rate } [\text{W/m}^3] = \frac{d^2 \times \Delta P^2}{16 \times l^2 \times \mu}$$

ΔP: pressure loss [Pa]
μ: viscosity [Pa·s]
1: pipe length [m]
d: pipe inner diameter [m]

(Measuring method for shear stress)

The viscosity (μ) of the cell suspension and the energy dissipation rate (ε) in Formula 2 are substituted into the following equation to obtain the shear stress.

$$\text{Shear stress } [\text{Pa}] = (\mu \times \varepsilon)^{0.5}$$

μ: viscosity [Pa·s]
ε: energy dissipation rate [W/m$^3$]

(Measuring method for LDH release rate)

The LDH release rate was calculated by the following formula.

$$\text{LDH release rate } [\%] = \frac{\text{LDH concentration of shear-loaded sample} - \text{LDH concentration of sample without shear load}}{\text{Concentration of LDH completely released} - \text{LDH concentration of sample without shear load}}$$

The LDH concentration was measured with a Cedex Bio manufactured by F. Hoffmann-La Roche, Ltd. The shear-loaded sample was subjected to centrifugation at 300 G-5 min after feeding the cell suspension into the micro flow passage, and the supernatant was measured. For the sample without shear load, the sample which have not been fed to the micro flow passage was centrifuged at 300 G for 5 minutes, and the supernatant was measured. For the sample that LDH was completely released, TWEEN (registered trademark) 20 was added to the sample that have not been fed into the micro flow passage, and vortexing of the mixture was continued for 30 minutes. Then, the mixture was centrifuged at 300 G for 5 minutes, and the supernatant was measured.

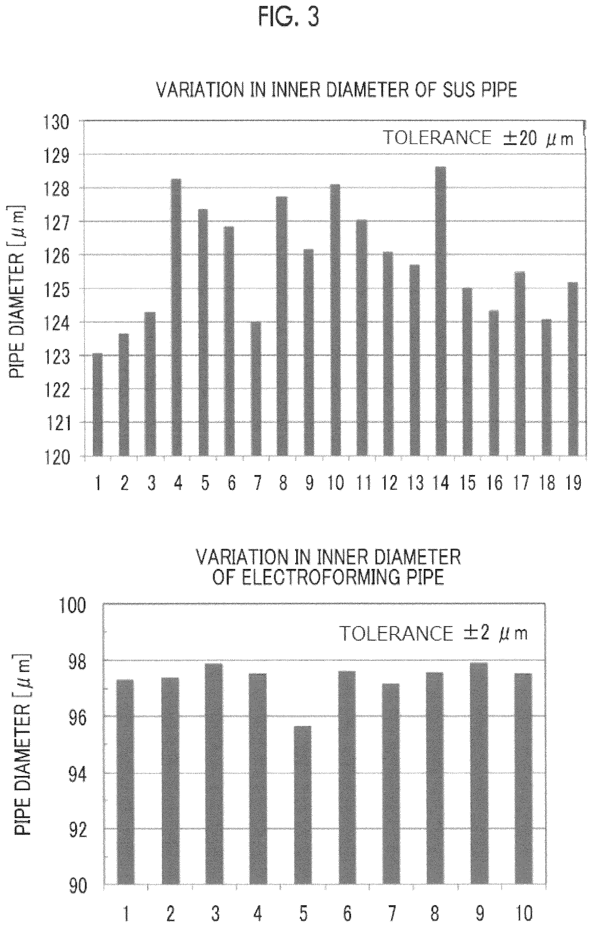

Fig. 3 shows the results of measuring the variation in the pipe inner diameter in a case where the pipe inner diameter for the SUS pipe and the electroforming pipe is calculated from the pressure loss in passing water. In the measurement, pure water is sucked with a syringe, and then the valve is switched to feed the liquid to the micro flow passage at 3.0 mL/min. The pipe inner diameter (d) was calculated by substituting the actually measured pressure loss (ΔP), viscosity (μ), pipe length (1), and flow rate (U) into the following equation.

$$\Delta P = \frac{128 \times \mu \times l \times U}{\pi \times d^4}$$

ΔP: pressure loss [Pa]

μ: viscosity [Pa·s]
1: pipe length [m]
U: flow rate [m$^3$/s]
d: pipe inner diameter [m]

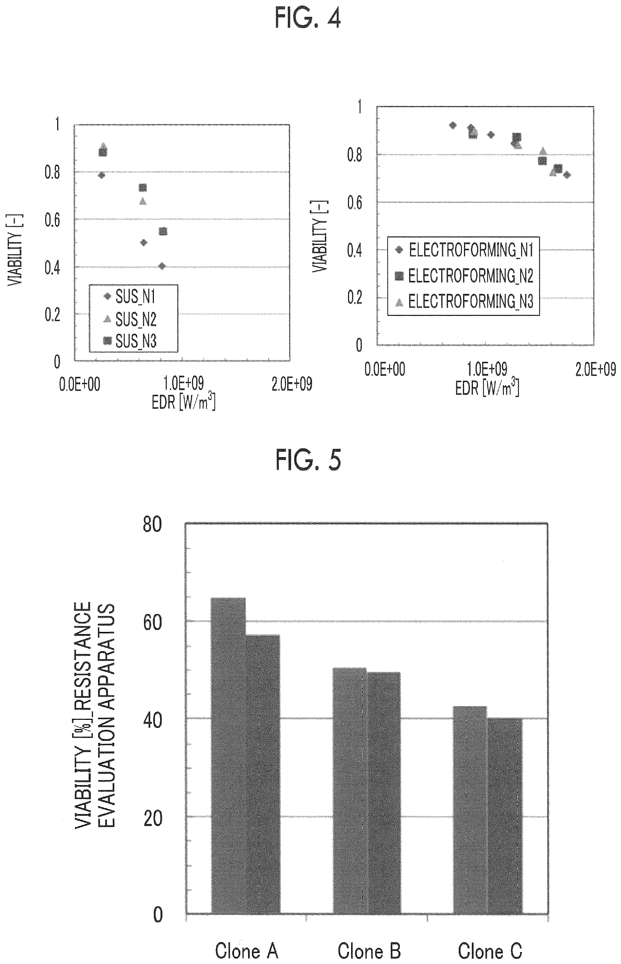

In addition, Fig. 4 and Table 1 show the results of measuring the repetitive reproducibility of the SUS pipe and the electroforming pipe using CHO cells. In the measurement, the cell suspension is sucked with a syringe, and then the valve is switched to feed the cell suspension to the micro flow passage. The viability was evaluated by gradually increasing the flow rate to 6.0, 9.0, and 10.0 [mL/min] at all levels of N3 at the level of the SUS pipe. At the level of the electroforming pipe, the viability was evaluated by gradually increasing the flow rate of N1 to 4.5, 5.0, 5.5, 6.0, and 7.0 [mL/min], and the flow rate of N2 and N3 to 5.0, 6.0, 6.5, and 6.8 [mL/min]. The N3 experiment was performed in each new micro flow passage. For the viability, the CV value of the SUS pipe was 7.7% to 19.0%, and the CV value of the electroforming pipe was 1.5% to 2.1%. It was found that the variation in the viability was smaller in the electroforming pipe, and the reproducibility was more excellent in a case where the measurement was performed using different pipes.

[Table 1]

| Type of pipe | EDR [W/m$^3$] | Viability [%] | | |
| --- | --- | --- | --- | --- |
| | | Average value | (Standard deviation | CV value |
| SUS | 2.7E+08 | 85.9% | 0.066 | 7.7% |
| | 6.4E+08 | 63.7% | 0.121 | 19.0% |
| Electroforming | 9.0E+08 | 89.9% | 0.014 | 1.5% |
| | 1.3E+09 | 85.3% | 0.018 | 2.1% |

The damage resistance evaluation value is quantified using the viable cell density of ViCell XR (the detection condition of the particles is 6 to 50 [μm] and the number of images is 50) manufactured by Beckman Coulter Life Sciences, as the ratio (viability) of the viable cell density after the damage loading to the viable cell density before the damage is loaded. To suppress the influence of variation after the filling operation of the cell into the syringe, the operation after "suction of the cell suspension with the syringe" was performed at N = 3 (three experiments), and to suppress the influence of the measurement variation of ViCellXR, ViCell measurement was performed three times for each experiment, and the viability was calculated. The damage resistance evaluation value of each experiment was calculated as the average value of the viability measured three times. Furthermore, a value obtained by averaging each damage resistance evaluation value (average value) of the three experiments was used for the viability in Fig. 5.

<2> Experimental results

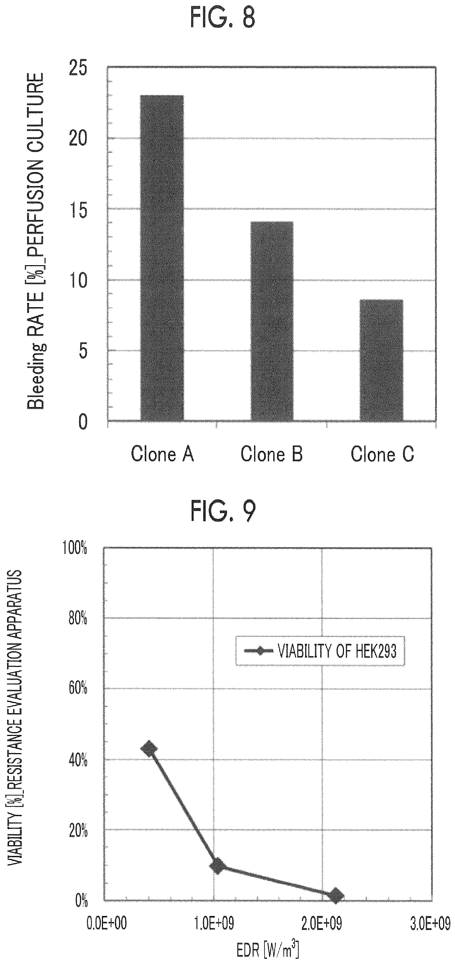

For three types of clones of the CHO cells, the quantification of the damage resistance evaluation value was performed twice according to the above procedure. The results are shown in Fig. 5.
Also for human embryonic kidney cell 293, the quantification of the damage resistance evaluation value was performed twice according to the above procedure. The results are shown in Fig. 9. As a condition for measuring HEK293 cells, the flow rate was gradually increased to 3.5, 5.5, and 7.7 [mL/min] at the level of the electroforming pipe (EDR in this case was $4.1 \times 10^8$, $1.0 \times 10^9$, and $2.1 \times 10^9$ [W/m$^3$]), and the viability was evaluated.
The order of resistance evaluation of CHO cells is clone A (64.9%, 57.4%) > clone B (50.4%, 49.5%) > clone C (42.7%, 40.2%), and the order of damage resistance of clone A, B, and C could be evaluated with good reproducibility.
It was found that the damage resistance of cell can be evaluated also for human embryonic kidney cell 293.

Example 2: Perfusion culture

<Method of perfusion culture>

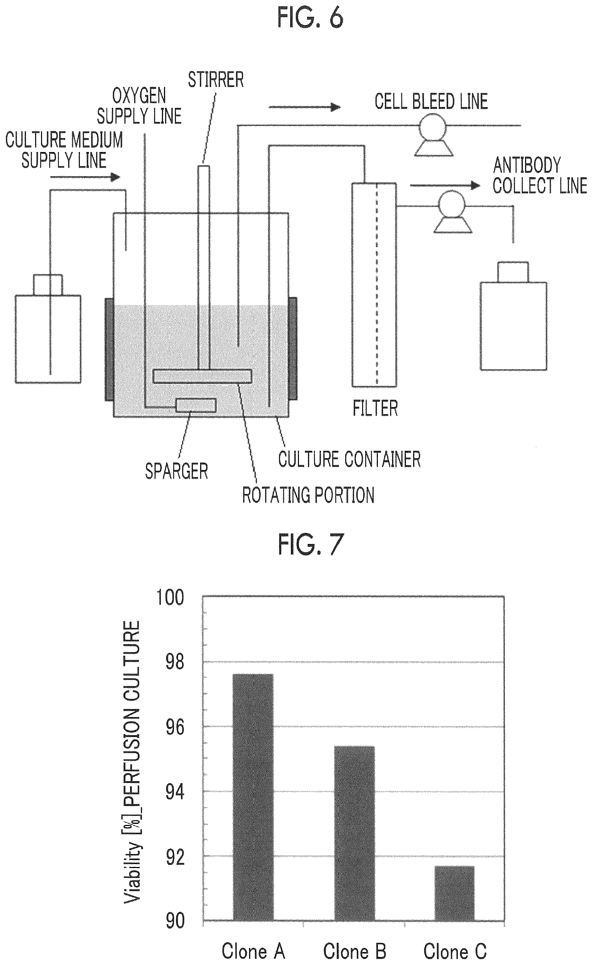

Cell culture was performed by a perfusion culture method using a cell culture apparatus having the configuration shown in Fig. 6. The cells used were monoclonal cells as described above. A glass container having a capacity of 2 L was used as the culture container, OptiCHO (Product No. 12681011) manufactured by Thermo Fisher Scientific, Inc. was used as the culture medium, and a hollow fiber membrane (F2 RF02PES) manufactured by Repligen Corporation was used as the filter for separating the cells and the antibody. The seeding density of the cells was $3 \times 10^5$ cells/mL,

and the liquid volume was 1 L. The supply of the fresh culture medium and the extraction of the cell suspension from the culture container were performed on or after the third day from the start of the culture. During the culture period, cell bleeding treatment was appropriately performed such that the cell density was maintained at about $120 \times 10^6$ cells/mL. The rotational speed of the rotating portion of the stirrer was set to 180 rpm from the start of the culture to the 10th day and 220 rpm after the 10th day. During the culture period, air having a flow rate of 0.1 L/min was supplied from the upper surface in the culture container. Pure oxygen was supplied from a 20 $\mu$m sparger disposed at the bottom of the culture container such that the oxygen concentration in the cell suspension within the culture container was 33%.

<Evaluation method for perfusion culture>

A sample was collected from the sampling port, and the viable cell density and the viability were measured with a ViCell XR manufactured by Beckman Coulter Life Sciences.

The viability in Fig. 7 is the ratio of the viable cell density to the total cell density (the sum of the viable cell density and the dead cell density) recognized on the ViCell. The bleeding in the present embodiment is an operation of pulling out excessively proliferated cells once a day to adjust a target cell density. The bleeding rate in Fig. 8 is the ratio of the cell suspension amount extracted by the bleeding operation described above to the total culture solution amount in the culture vessel. The higher the proliferation rate of the cells is, the higher the bleeding rate is.

<Result of perfusion culture>

The measurement result of the viability in the perfusion culture is shown in Fig. 7. In a case where the perfusion culture of clones A, B, and C were performed on a 1 L scale, the average value of viabilities from Day 10 to Day 52 was clone A (97.6%) > clone B (95.4%) > clone C (91.7%).

In addition, the measurement result of the Bleeding rate, which is an index of the cell proliferation rate in the perfusion culture is shown in Fig. 8. The average value of the bleeding rates from Day 10 to Day 52 was clone A (23.0%) > clone B (14.1%) > clone C (8.6%), which was the same tendency as the order of damage resistance evaluation.

**Claims**

1. A method for selecting cells, comprising:

   introducing cells into a micro flow passage and allowing the cells to pass through the micro flow passage;
   evaluating damage resistance of the cells to damage received by the cells due to passing through the micro flow passage; and
   selecting cells based on the evaluation of the damage resistance.

2. The method according to claim 1,
   wherein the damage received by the cell is stress received from a fluid.

3. The method according to claim 1 or 2,
   wherein the damage resistance of the cell is evaluated based on a damage state of the cell.

4. The method according to any one of claims 1 to 3,
   wherein the damage resistance is evaluated based on viability with respect to an energy dissipation rate.

5. The method according to any one of claims 1 to 3,
   wherein the damage resistance is evaluated based on a lactate dehydrogenase release rate of the cell with respect to an energy dissipation rate.

6. The method according to any one of claims 1 to 3,
   wherein the damage resistance is evaluated based on viability against shear stress.

7. The method according to any one of claims 1 to 3,
   wherein the damage resistance is evaluated based on a lactate dehydrogenase release rate of the cell against shear stress.

8. The method according to any one of claims 1 to 7,

wherein a plurality of the micro flow passages are used, and the micro flow passages are exchanged to evaluate the damage resistance of the cell.

9. The method according to claim 8,
wherein an inner diameter dimensional tolerance in the plurality of the micro flow passages is less than ±10%.

10. The method according to any one of claims 1 to 9,
wherein the micro flow passage is an electroforming tube.

11. The method according to any one of claims 1 to 10,
wherein an inner diameter of the micro flow passage is 10 $\mu$m to 3000 $\mu$m.

12. The method according to any one of claims 1 to 7,
wherein the micro flow passage is a groove.

13. The method according to claim 12,
wherein the groove consists of a combination of a metal plate and a metal plate with a groove.

14. The method according to claim 12 or 13,
wherein a width of each side of a cross section of the groove is 10 $\mu$m to 3000 $\mu$m.

15. The method according to any one of claims 1 to 14,
wherein the cell is a Chinese hamster ovary-derived cell or a human embryonic kidney cell 293.

16. The method according to any one of claims 1 to 15,
wherein the cells to be selected are a cell for producing a protein.

17. The method according to any one of claims 1 to 16,
wherein the cells are cells obtained by increasing the number of monoclonal cells.

18. The method according to claim 16 or 17,
wherein the cell for producing a protein is a cell for producing a protein by perfusion culture.

19. An apparatus for selecting cells, comprising:

a micro flow passage;
a pressure gauge for measuring a pressure upstream of the micro flow passage;
a first pump for feeding a cell suspension, which is provided upstream of the micro flow passage;
a second pump for feeding a cleaning liquid, which is provided upstream of the micro flow passage;
a pipe for supplying the cell suspension to the first pump and a pipe for supplying the cleaning liquid to the second pump;
a pipe for supplying a liquid from the first pump and a liquid from the second pump to the micro flow passage; and
a pipe for collecting liquid discharged from the micro flow passage.

20. An apparatus system for selecting cells, comprising:

the apparatus for selecting cells according to claim 19; and
a plurality of micro flow passages for being exchangeably used.

21. The apparatus system according to claim 20,
wherein an inner diameter dimensional tolerance in the plurality of the micro flow passages is less than ±10%.

# FIG. 1

LIQUID FEED PUMP
FOR CLEANING LIQUID

MICRO FLOW
PASSAGE

FEEDING LIQUID
INTO MICRO
FLOW PASSAGE

PRESSURE
SENSOR

FILLING CELL
SUSPENSION

LIQUID FEED PUMP
FOR CELL SUSPENSION

CELL SUSPENSION

# FIG. 2

BASE PLATE
(WITHOUT GROOVE)

BASE PLATE
(WITH GROOVE)

BASE PLATE
(FITTED SURFACES)

MICRO FLOW
PASSAGE

MICRO FLOW
PASSAGE

## FIG. 3

### VARIATION IN INNER DIAMETER OF SUS PIPE

TOLERANCE ±20 $\mu$m

### VARIATION IN INNER DIAMETER OF ELECTROFORMING PIPE

TOLERANCE ±2 $\mu$m

# FIG. 4

# FIG. 5

# FIG. 6

# FIG. 7

## FIG. 8

## FIG. 9

## INTERNATIONAL SEARCH REPORT

| International application No. |
| --- |
| **PCT/JP2022/000292** |

### A. CLASSIFICATION OF SUBJECT MATTER

*C12M 1/00*(2006.01)i; *C12M 1/34*(2006.01)i; *C12M 3/00*(2006.01)i; *C12N 5/10*(2006.01)i; *C12Q 1/04*(2006.01)i;
*C12Q 1/32*(2006.01)i
FI: C12Q1/04; C12M1/00 A; C12M1/34 B; C12M3/00 Z; C12Q1/32; C12N5/10

According to International Patent Classification (IPC) or to both national classification and IPC

### B. FIELDS SEARCHED

Minimum documentation searched (classification system followed by classification symbols)

C12M1/00; C12M1/34; C12M3/00; C12N5/10; C12Q1/04; C12Q1/32

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | WO 2020/132231 A1 (GENENTECH, INC.) 25 June 2020 (2020-06-25) claims, paragraphs [0127], [0156], [0166], fig. 1 | 1-21 |
| Y | claims, paragraphs [0127], [0156], [0166], fig. 1 | 1-21 |
| X | ARENA, Tia A. et al. An anti-apoptotic HEK293 cell line provides a robust and high titer platform for transient protein expression in bioreactors. mAbs. 2019, vol. 11, no. 5, pp. 977-986 abstract, p. 978, left column, paragraph [0003] to right column, paragraph [0001], p. 983, right column, paragraph [0004] | 1-21 |
| Y | abstract, p. 978, left column, paragraph [0003] to right column, paragraph [0001], p. 983, right column, paragraph [0004] | 1-21 |
| Y | MOLLET, Mike et al. Acute Hydrodynamic Forces and Apoptosis: A Complex Question. Biotechnology and Bioengineering. 2007, vol. 98, no. 4, pp. 772-788 abstract, p. 774, left column, paragraph [0003], fig. 2 | 1-21 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **24 February 2022** | **08 March 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2022/000292** |

**C.      DOCUMENTS CONSIDERED TO BE RELEVANT**

| Category* | Citation of document, with indication, where appropriate, of the relevant passages | Relevant to claim No. |
| --- | --- | --- |
| Y | MA, Ningning et al. Fabrication and Use of a Transient Contractional Flow Device to Quantify the Sensitivity of Mammalian and Insect Cells to Hydrodynamic Forces. Biotechnology and Bioengineering. 2002, vol. 80, no. 4, pp. 428-437<br>abstract, fig. 1 | 1-21 |

Form PCT/ISA/210 (second sheet) (January 2015)

INTERNATIONAL SEARCH REPORT
Information on patent family members

International application No.

**PCT/JP2022/000292**

| Patent document cited in search report | | | Publication date (day/month/year) | Patent family member(s) | | | Publication date (day/month/year) |
|---|---|---|---|---|---|---|---|
| WO | 2020/132231 | A1 | 25 June 2020 | US | 2021/0317501 | A1 | |
| | | | | JP | 2022-514082 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016052335 A

- JP 2016517691 A

**Non-patent literature cited in the description**

- **ADRIAN JOSEPH et al.** *Biotechnol. J.,* 2017, vol. 12, 1600730